# EUROPEAN PATENT APPLICATION

(11) **EP 4 275 726 A1**
(43) Date of publication of application: **15.11.2023**
(21) Application number: 22739342.8
(22) Date of filing: 07.01.2022
(51) Int. Cl.: A61M 11/06, A61M 5/315

(54) **SPRAYER**

(30) Priority: 14.01.2021 JP 2021004063
(71) Applicant: Nipro Corporation, Osaka-shi, Osaka 531-8510 (JP)
(72) Inventor: YABE, Yukihiro, Osaka-shi, Osaka 531-8510 (JP); HIROBE, Teruhisa, Osaka-shi, Osaka 531-8510 (JP)
(74) Representative: Prüfer & Partner mbB Patentanwälte · Rechtsanwälte
(86) International application number: PCT/JP2022/000330
(87) International publication number: WO 2022/153933

(57) **Abstract**

A sprayer (1) includes a barrel (200), a finger grip (300), a gasket (400), a rod (500), and a plunger (700) slidably attached to the rod (500) and capable of contacting the rod (500) and pressing the rod (500) toward a distal side. The plunger (700) includes a contact member (710) which is brought into contact with the finger grip (300), and a first pressing surface (720) provided at a proximal end thereof. The rod (500) includes a second pressing surface (550) provided at a proximal end thereof. The contact member (710) is brought into contact with the finger grip (300) to restrict the movement of the plunger (700) toward the distal side with the gasket (400) positioned between a distal end (270) and a proximal end (280) of the barrel (200).

## Description

### TECHNICAL FIELD

The present invention relates to a sprayer, and more particularly to an instrument for administering a drug by spraying.

### BACKGROUND ART

There are various methods of administering drugs, one of which is nasal administration. The nasal administration is such a method that each nasal cavity is usually sprayed with the same amount of drug. The nasal administration has an advantage that it does not cause pain like subcutaneous injection.

In the nasal administration, since the drug is sprayed into each nasal cavity, two times of drug administrations are required. For example, PTL 1 (WO2012/002398) describes such a sprayer that enables two times of drug administrations with a single sprayer. In the sprayer described in PTL 1, after the first drug administration, the second drug administration is possible by rotating the rod.

### CITATION LIST

### PATENT LITERATURE

[PTL 1]: WO2012/002398

### SUMMARY OF INVENTION

### TECHNICAL PROBLEM

In the sprayer described in PTL 1, after the first drug administration, it is necessary to rotate the rod to perform the second drug administration. In order to rotate the rod, the operator needs to hold the barrel containing the drug solution with one hand and rotate the rod with the other hand. Therefore, after the first drug administration, the operator needs to change the way of holding the sprayer and operate the sprayer with both hands.

The present disclosure has been made in order to solve the above-mentioned problem, and an object of the present disclosure is to provide a sprayer which allows an operator to perform a second drug administration without having to change the way of holding the sprayer after a first drug administration.

### SOLUTION TO PROBLEM

A sprayer according to the present disclosure includes: a spray nozzle for spraying a drug solution; a barrel having a distal end, a proximal end, and a cavity extending along an axis connecting the distal end and the proximal end, the spray nozzle being attached to the distal end; a finger grip provided at the proximal end of the barrel; a gasket disposed inside the barrel and slidable inside the barrel; a rod provided at a proximal end of the gasket and having a distal end inserted into the cavity of the barrel; and a plunger slidably attached to the rod and capable of contacting the rod and pressing the rod toward a distal side. The plunger includes a contact member which is brought into contact with the finger grip, and a first pressing surface provided at a proximal end thereof. The rod includes a second pressing surface provided at a proximal end thereof. The contact member is brought into contact with the finger grip to restrict the movement of the plunger toward the distal side with the gasket positioned between the distal end and the proximal end of the barrel.

In the sprayer, at least one of the first pressing surface and the second pressing surface may be marked with an order for an operator to press.

In the sprayer, the first pressing surface and the second pressing surface may be provided close to each other, and an area of the second pressing surface may be larger than an area of the first pressing surface.

In the sprayer, the first pressing surface may be positioned closer to a proximal side than the second pressing surface.

### ADVANTAGEOUS EFFECTS OF INVENTION

According to the present disclosure, it is possible to provide a sprayer which allows an operator to perform a second drug administration without having to change the way of holding the sprayer after the first drug administration.

### BRIEF DESCRIPTION OF DRAWINGS

Fig. 1 is a perspective view of a sprayer according to the present embodiment before a first drug administration;
Fig. 2 is a front view of the sprayer according to the present embodiment before the first drug administration;
Fig. 3 is an exploded perspective view of the sprayer according to the present embodiment;
Fig. 4 is a perspective view of a finger grip according to the present embodiment;
Fig. 5 is a perspective view of a rod according to the present embodiment;
Fig. 6 is a perspective view of a plunger according to the present embodiment;
Fig. 7 is a perspective view of the sprayer according to the present embodiment viewed from the proximal end before the first drug administration;
Fig. 8 is a front view of the sprayer according to the present embodiment after the first drug administration; and
Fig. 9 is a front view of the sprayer according to the present embodiment after a second drug administration.

### DESCRIPTION OF EMBODIMENTS

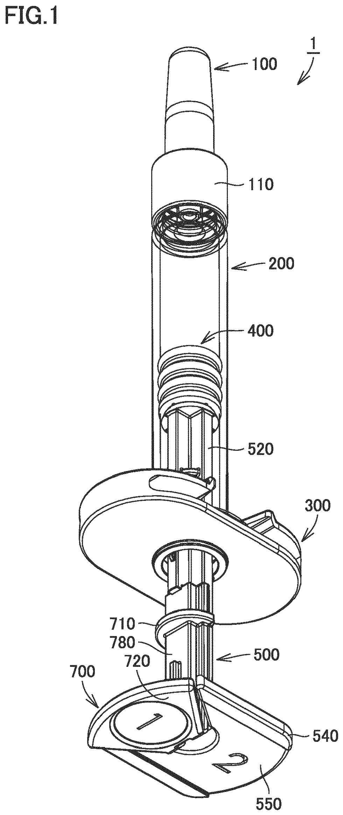

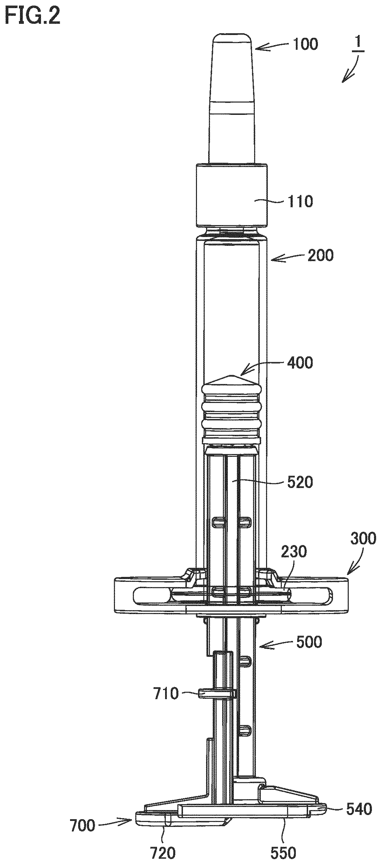

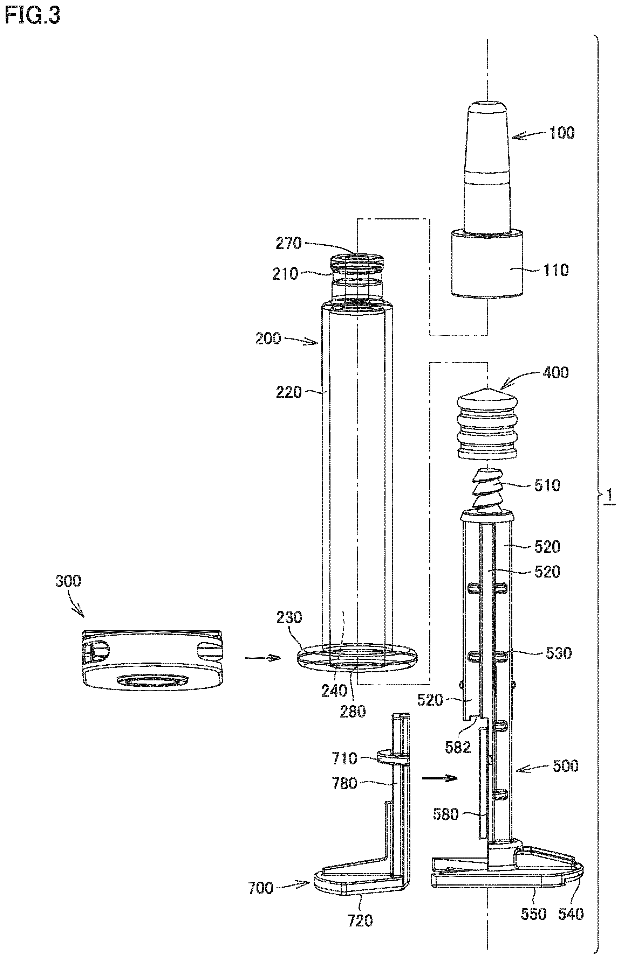

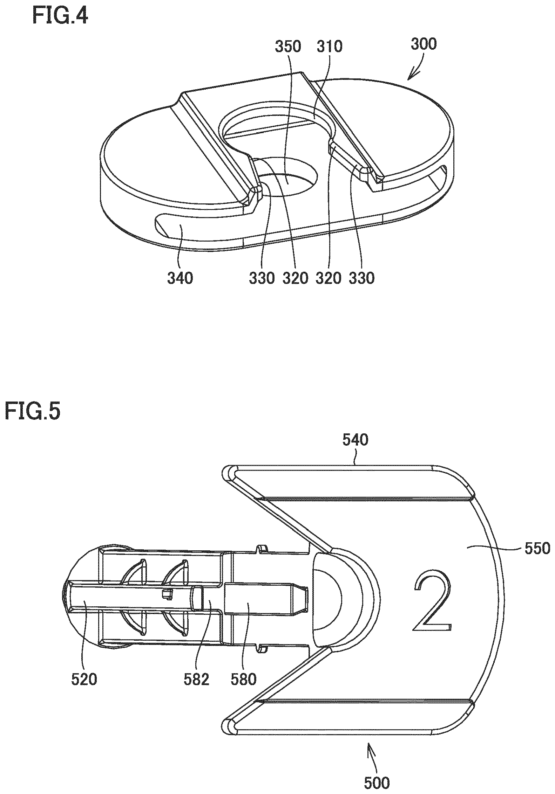

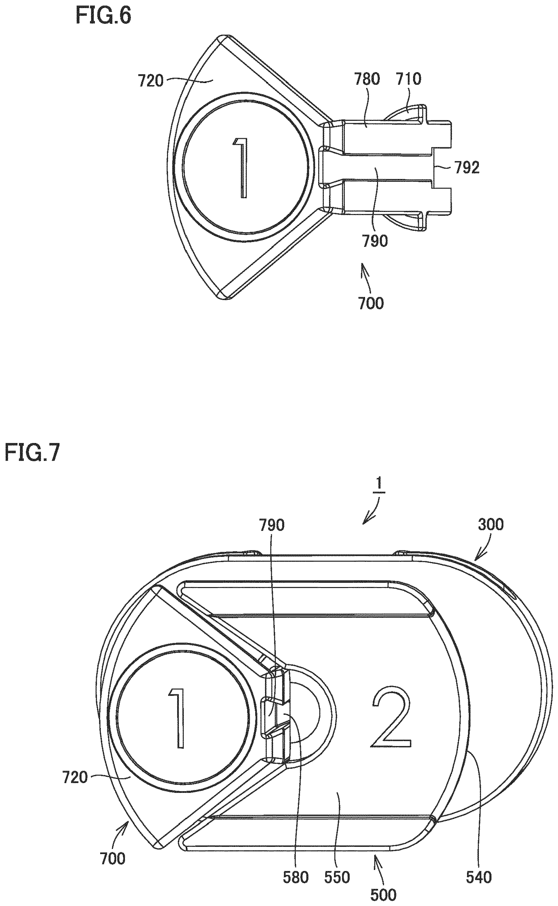

Hereinafter, an embodiment of a sprayer according to the present disclosure will be described in detail with reference to the drawings. Fig. 1 is a perspective view of a sprayer according to the present embodiment before a first drug administration. Fig. 2 is a front view of the sprayer according to the present embodiment before the first drug administration. Fig. 3 is an exploded perspective view of the sprayer according to the present embodiment. Fig. 4 is a perspective view of a finger grip according to the present embodiment. Fig. 5 is a perspective view of a rod according to the present embodiment. Fig. 6 is a perspective view of a plunger according to the present embodiment. Fig. 7 is a perspective view of the sprayer according to the present embodiment viewed from the proximal end before the first drug administration.

The sprayer according to the present embodiment is a sprayer having a two-stage administration mechanism for administering a drug solution to each of a pair of nasal cavities.

The sprayer 1 according to the present embodiment includes a spray nozzle 100, a barrel 200, a finger grip 300, a gasket 400, a rod 500, and a plunger 700. In the following description, the front end side of the sprayer 1 where the spray nozzle 100 is located is defined as a distal side, and the rear end side of the sprayer 1 where the rod 500 is located is defined as a proximal side.

The spray nozzle 100 is provided with a spray hole at a distal end thereof to spray a drug solution. A nozzle joint 110 is provided at a proximal end of the spray nozzle 100. The nozzle joint 110 is connected to a barrel joint 210 provided at the distal end of the barrel 200 by fitting.

A pressure applying member (not shown) is provided inside the spray nozzle 100 to increase the pressure of the drug solution in the barrel 200 and apply a spray force to the drug solution. The pressure applying member has a flow path that gradually narrows, thereby increasing the pressure of the drug solution transferred to the spray nozzle 100.

The barrel 200 has a distal end 270 and a proximal end 280. The spray nozzle 100 is attached to the distal end 270 of the barrel 200. The barrel 200 has a cavity 240 extending along an axis connecting the distal end 270 and the proximal end 280 (hereinafter referred to as a "barrel central axis"). The cavity 240 is a cylindrical space. The drug solution (not shown) is stored in the cavity 240.

The barrel joint 210 is provided at the distal end 270 of the barrel 200. The nozzle joint 110 of the spray nozzle 100 is connected to the barrel joint 210. Thus, the spray nozzle 100 is connected to the barrel 200.

The proximal end 280 of the barrel 200 is provided with a disk-shaped flange 230. The flange 230 protrudes from the barrel body 220 in a direction away from the barrel central axis in a plane orthogonal to the barrel central axis.

A finger grip 300, which is a separate member in the present embodiment, is fitted on the flange 230. The finger grip is a protrusion to be gripped by the operator with his/her fingers when the operator performs a drug administration. Instead of providing the finger grip 300 as a separate member as in the present embodiment, the flange 230 itself may be used as a finger grip.

The finger grip 300 has an oval planar shape in the present embodiment. The planar shape of the finger grip 300 may be any other shape as long as it can by gripped by the operator with his/her fingers. For example, the planar shape of the finger grip 300 may be circular, elliptical, or rectangular.

A distal end surface of the finger grip 300 is provided with a substantially circular notch 310. The proximal end of the barrel body 220 is inserted into the notch 310. The notch 310 is open toward one side surface of the finger grip 300.

The opposing side surfaces of the notch 310 are provided with a pair of barrel holding members 320 configured to hold the proximal end of the inserted barrel body 220. The barrel holding members 320 are provided opposing to each other, and the distance between the barrel holding members 320 is smaller than the outer diameter of the barrel body 220.

The finger grip 300 is provided with a pair of inclined surfaces 330 that lead to the barrel holding member 320 for guiding the barrel body 220. The distance between the inclined surfaces 330 increases toward the side surface of the finger grip 300. By inserting the barrel body 220 along the inclined surfaces 330, the finger grip 300 can be easily attached to the barrel 200.

The finger grip 300 is provided with a slit 340 into which the flange 230 is inserted. By inserting the flange 230 into the slit 340, the finger grip 300 attached to the barrel 200 is prevented from deviating from the direction of the barrel central axis.

A proximal end surface of the finger grip 300 is provided with a through hole 350. The through hole 350 and the notch 310 are provided in such a manner that the central axes thereof overlap each other. The rod 500 is inserted through the through hole 350.

A gasket 400 is provided in the cavity 240 of the barrel 200. The gasket 400 can slide in the cavity 240 of the barrel 200 while maintaining liquid tightness. The materials of the barrel 200 and the gasket 400 are selected in such a manner that they do not react with the drug solution stored in the barrel 200 and allow the gasket 400 to slide inside the barrel 200 while maintaining liquid tightness. Example materials of the gasket 400 include thermoplastic elastomer and rubber.

The gasket 400 is provided with a threaded hole. A screw 510 provided at the distal end of the rod 500 is screwed into the threaded hole. Thereby, the gasket 400 is fixed to the distal end of the rod 500. The gasket 400 and the rod 500 may be integrally molded from synthetic resin or the like.

The rod 500 is provided at the proximal end of the gasket 400, and the gasket 400 and the distal end of the rod 500 are inserted into the cavity 240 of the barrel 200. The rod 500 has a proximal rib 540 provided at the proximal end of the rod 500.

The rod 500 has a rod body which extends along an axis (hereinafter, will be referred to as a "rod central axis") that overlaps the barrel central axis when being inserted into the barrel 200. The rod body is constituted by four first reinforcing ribs 520 extending along the rod central axis. The rod body has a cross section constituted by four first reinforcing ribs 520. The center of the cross section coincides with the rod central axis. A second reinforcing rib 530 is provided between a pair of first reinforcing ribs 520 adjacent to each other at an angle of 90°. The second reinforcing ribs 530 are provided at a plurality of positions in the direction of the rod central axis.

A part of the rod 500 close to the proximal end thereof is provided with an engagement rail 580 instead of the first reinforcing rib 520. The engagement rail 580 is provided along the rod body and extends along the rod central axis. The engagement rail 580 engages with an engagement groove 790 provided on the plunger 700, which will be described later. Thus, the rod 500 holds the plunger 700 in such a manner that the plunger 700 is slidable in the direction of the rod central axis.

A contact surface 582 is provided facing the distal end of the engagement rail 580. The contact surface 582 is formed by the proximal end surface of the first reinforcing rib 520. The distal end surface of the plunger 700 is in contact with the contact surface 582. The pressing force for pressing the plunger 700 toward the distal side is transmitted to the rod 500 via the contact surface 582. Thus, the rod 500 can be moved toward the distal side by pressing the plunger 700.

The screw 510 is provided on the distal end of the rod 500. A proximal rib 540 is provided on the proximal end of the rod 500. The proximal end surface of the proximal rib 540 constitutes a second pressing surface 550 for the operator to press the rod 500 toward the distal side. In the present embodiment, the second pressing surface 550 is flat. The second pressing surface 550 is marked with a number "2" indicating an order for the operator to press the second pressing surface. According to this indication, the operator can press the second pressing surface 550 of the rod 500 in the second drug administration. In the second drug administration, the operator presses only the second pressing surface 550.

A first pressing surface 720, which will be described later, is provided in line with the second pressing surface 550. As illustrated in Fig. 5, the second pressing surface 550 is provided with a depression to receive the first pressing surface 720. The depression of the second pressing surface 550 has a substantially trapezoidal shape as illustrated in Fig. 5. The recess may have a semicircular shape, a triangular shape, or the like.

The plunger 700 is slidably attached to the rod 500. More specifically, the plunger 700 is slidably attached to the rod 500 in the direction of the rod central axis by the engagement between the engagement rail 580 of the rod 500 and the engagement groove 790 of the plunger 700.

In the present embodiment, the rod 500 is provided with an engagement rail 580, and the plunger 700 is provided with an engagement groove 790. Alternatively, the rod 500 may be provided with an engagement groove, and the plunger 700 may be provided with an engagement rail. Further, as long as the plunger 700 can linearly move relative to the rod 500, the engagement is not limited to an engagement groove and an engagement rail. For example, one of the rod 500 and the plunger 700 may be provided with an engagement groove, and the other one of the rod 500 and the plunger 700 may be provided with a plurality of protrusions (for example, two protrusions) that engage with the engagement groove to allow the plunger 700 to slide relative to the rod 500.

The plunger 700 has a contact member 710 that is brought into contact with the finger grip 300. The contact member 710 is brought into contact with the peripheral edge of the through hole 350 provided on the finger grip 300. The contact member 710 is formed as a semicircular flange. The contact member 710 protrudes in a direction orthogonal to the rod central axis. The contact member 710 may have any shape as long as it can contact the peripheral edge of the through hole 350.

The first pressing surface 720 is provided on the proximal end of the plunger 700. The first pressing surface 720 extends along a plane orthogonal to the rod central axis. As illustrated in Fig. 6, the first pressing surface 720 has a substantially fan shape. As illustrated in Fig. 7, the first pressing surface 720 fits into the depression provided on the second pressing surface 550 of the rod 500. In other words, the first pressing surface 720 and the second pressing surface 550 are disposed close to each other. The shape of the first pressing surface 720 may be modified according to the shape of the depression of the first pressing surface 720.

As illustrated in Fig. 7, the area of the second pressing surface 550 is larger than the area of the first pressing surface 720. In the first drug administration, both the first pressing surface 720 and the second pressing surface 550 may be pressed, whereas in the second drug administration, only the second pressing surface 550 needs to be pressed. Since the area of the second pressing surface 550 is larger than the area of the first pressing surface 720, it is easy to selectively press only the second pressing surface 550 in the second drug administration. The area of the first pressing surface 720 and the area of the second pressing surface 550 may be the same, or the area of the first pressing surface 720 may be larger than the area of the second pressing surface 550.

As illustrated in Figs. 1 and 2, when the distal end of the plunger 700 is in contact with the contact surface 582, the first pressing surface 720 is located closer to the proximal side than the second pressing surface 550. The first pressing surface 720 is marked with a number " 1" indicating an order for the operator to press the first pressing surface. According to this indication, the operator can press the first pressing surface 720 of the plunger 700 in the first drug administration. Since pressing the first pressing surface 720 of the plunger 700 also moves the rod 500, the operator may press the first pressing surface 720 and the second pressing surface 550 simultaneously.

In the present embodiment, the first pressing surface 720 is marked with a number "1" and the second pressing surface 550 is marked with a number "2", but different indications may be used to indicate the order. For example, the first pressing surface 720 may be marked with a letter "A", and the second pressing surface 550 may be marked with a letter "B", which can be understood by the operator as an order.

The pressing order may be marked only on one of the first pressing surface 720 and the second pressing surface 550. For example, the first pressing surface 720 may be marked with an arrow pointing toward the second pressing surface 550.

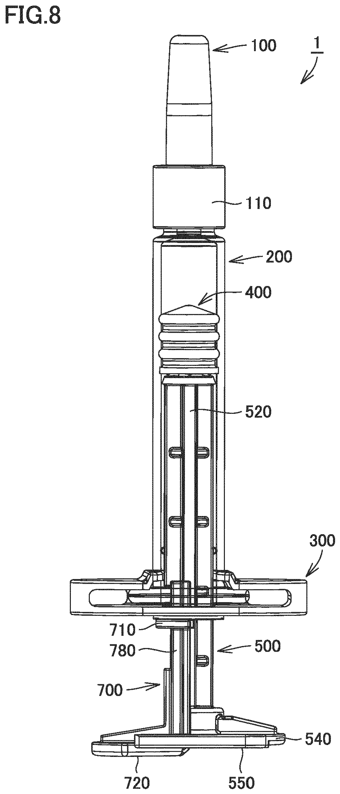

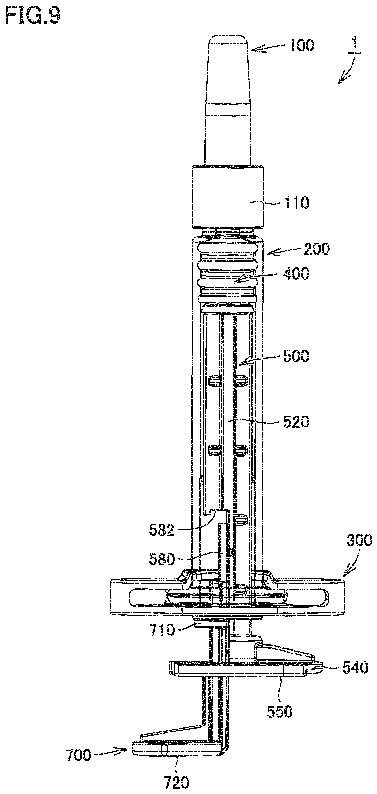

Next, the operations of the sprayer 1 of the present embodiment will be described with reference to Figs. 2, 8 and 9. Figs. 2, 8 and 9 are front views of the sprayer according to the present embodiment. Fig. 2 illustrates the sprayer in a state before the first drug administration, Fig. 8 illustrates the sprayer in a state after the first drug administration, and Fig. 9 illustrates the sprayer in a state after the second drug administration.

As illustrated in Fig. 2, the sprayer 1 according to the present embodiment includes a spray nozzle 100, a barrel 200, a finger grip 300, a gasket 400, a rod 500, and a plunger 700.

The spray nozzle 100 is attached to the distal end 270 of the barrel 200. The finger grip 300 is attached to the proximal end 280 of the barrel 200. The gasket 400 is attached to the distal end of the rod 500. The drug solution is stored in the cavity 240 of the barrel 200. The space between the gasket 400 and the distal end 270 of the barrel 200 is filled with the drug solution.

The plunger 700 is slidably attached to the rod 500. The distal end of the rod 500 and the gasket 400 are inserted into the cavity 240 of the barrel 200.

The operator performs a priming operation prior to the state illustrated in Fig. 2. The priming operation is an operation to fill the spray nozzle 100 to the distal end thereof with a drug solution to make it ready for the first drug administration. Specifically, the operator presses the rod 500 and the plunger 700 toward the distal side to ensure that air is discharged from the spray nozzle 100. Fig. 2 illustrates a state in which the priming operation has been completed.

In the state illustrated in Fig. 2, the operator inserts the spray nozzle 100 into one nasal cavity and performs the first drug administration. Specifically, the operator grips the finger grip 300 with the index finger and the middle finger, and presses the first pressing surface 720 of the plunger 700 and the second pressing surface 550 of the rod 500 with the thumb. The operator may press only the first pressing surface 720. As a result, the plunger 700, the rod 500 and the gasket 400 are moved toward the distal side, and the drug solution stored in the barrel 200 is sprayed from the spray nozzle 100 in an atomized form.

As illustrated in Fig. 8, when the contact member 710 of the plunger 700 is brought into contact with the finger grip 300, the first drug administration is completed. When the contact member 710 is brought into contact with the finger grip 300, the further movement of the plunger 700 toward the distal side is restricted.

Next, the operator shifts the thumb pressing the first pressing surface 720 and the second pressing surface 550, and places the thumb only on the second pressing surface 550. When advancing from the first drug administration to the second drug administration, the operator does not need to change the way of holding the sprayer 1, which makes it possible to easily advance from the first drug administration to the second drug administration.

In this state, the operator inserts the spray nozzle 100 into the other nasal cavity and performs the second drug administration. Specifically, the operator grips the finger grip 300 with the index finger and the middle finger, and presses only the second pressing surface 550 of the rod 500 with the thumb. Since the plunger 700 is slidably attached to the rod 500, the plunger 700 slides relative to the rod 500. Thus, while the plunger 700 is kept at the position where the first drug administration is completed, the rod 500 and the gasket 400 are further moved toward the distal side, and thereby, the remaining drug solution in the barrel 200 is sprayed from the spray nozzle 100 in an atomized state. Fig. 9 illustrates a state after the second drug administration is completed. When the second drug administration is completed, the gasket 400 reaches the distal end of the cavity of the barrel 200.

By performing the drug administration in this manner, it is possible to perform the first drug administration while leaving a necessary amount of the drug solution in the barrel 200 for the second drug administration.

The amount of the drug solution jetted by advancing the gasket 400 from the position of the gasket 400 at the end of the priming operation illustrated in Fig. 2 to the position of the gasket 400 at the end of the first drug administration illustrated in Fig. 8 is equal to the amount of the drug solution to be administered in the first drug administration. Further, the amount of the drug solution jetted by advancing the gasket 400 from the position of the gasket 400 at the end of the first drug administration illustrated in Fig. 8 to the position of the gasket 400 at the end of the second drug administration illustrated in Fig. 9 is equal to the amount of the drug solution to be administered in the second drug administration.

To equalize the amount of the drug solution to be administered in the first drug administration and the amount of the drug solution to be administered in the second drug administration, for example, the distance between the finger grip 300 and the contact member 710 of the plunger 700 in the state before the first drug administration illustrated in Fig. 2 may be adjusted.

The amount of the drug solution to be administered in the second drug administration is determined by the dimensions of the sprayer 1 such as the plunger 700, the barrel 200 and the like. The dimensions of the sprayer 1 are determined according to the amount of the drug solution required in the second drug administration.

As described above, the sprayer 1 according to the present disclosure includes: a spray nozzle 100 for spraying a drug solution; a barrel 200 having a distal end 270, a proximal end 280, and a cavity 240 extending along an axis connecting the distal end 270 and the proximal end 280, the spray nozzle 100 being attached to the distal end 270; a finger grip 300 provided at the proximal end 280 of the barrel 200; a gasket 400 disposed inside the barrel 200 and slidable inside the barrel 200; a rod 500 provided at the proximal end of the gasket 400 and having a distal end inserted into the cavity 240 of the barrel 200; and a plunger 700 slidably attached to the rod 500 and capable of contacting the rod 500 and pressing the rod 500 toward the distal side. The plunger 700 includes a contact member 710 which is brought into contact with the finger grip 300, and a first pressing surface 720 provided at a proximal end thereof. The rod 500 has a second pressing surface 550 at a proximal end thereof. The contact member 710 is brought into contact with the finger grip 300 to restrict the movement of the plunger 700 toward the distal side with the gasket 400 positioned between the distal end 270 and the proximal end 280 of the barrel 200.

In the sprayer 1, the first drug administration can be performed by pressing the first pressing surface 720 of the plunger 700 until the contact member 710 of the plunger 700 is brought into contact with the finger grip 300. The operator can perform the second drug administration by pressing the second pressing surface 550 without changing the way of holding the sprayer 1.

In the sprayer 1, at least one of the first pressing surface 720 and the second pressing surface 550 is marked with an order for the operator to press. Since the pressing surface is marked with an order, the operator can press the first pressing surface 720 and then press the second pressing surface 550 according to the marked order.

In the sprayer 1, the first pressing surface 720 and the second pressing surface 550 are provided close to each other, and the area of the second pressing surface 550 is larger than the area of the first pressing surface 720.

In the first drug administration, both the first pressing surface 720 and the second pressing surface 550 are pressed, whereas in the second drug administration, only the second pressing surface 550 needs to be pressed. By providing the first pressing surface 720 and the second pressing surface 550 close to each other, it is easy to press both the first pressing surface 720 and the second pressing surface 550 in the first drug administration. Further, since the area of the second pressing surface 550 is larger than the area of the first pressing surface 720, it is easy to selectively press only the second pressing surface 550 in the second drug administration.

In the sprayer 1, the first pressing surface 720 is positioned closer to the proximal side than the second pressing surface 550.

In the sprayer 1, since the first pressing surface 720 is positioned closer to the proximal side than the second pressing surface 550, the operator first touches the first pressing surface 720 in the first drug administration, which makes it possible to reduce an operation error in which the operator presses only the second pressing surface 550 in the first drug administration.

It should be understood that the embodiments disclosed herein are illustrative and non-restrictive in all respects. The scope of the present invention is defined by the terms of the claims rather than the description of the embodiments above, and is intended to include any modifications within the scope and meaning equivalent to the terms of the claims.

### REFERENCE SIGNS LIST

1: sprayer; 100: spray nozzle; 110: nozzle joint; 200: barrel; 210: barrel joint; 220: barrel body; 230: flange; 240: cavity; 270: distal end; 280: proximal end; 300: finger grip; 310: notch; 320: barrel holding member; 330: inclined surface; 340: slit; 350: through hole; 400: gasket; 500: rod; 510: screw; 520: first reinforcing rib; 530: second reinforcing rib; 540: proximal rib; 550: second pressing surface; 580: engagement rail; 582: contact surface; 700: plunger; 710: contact member; 720: first pressing surface; 790: engagement groove

## Claims

1. A sprayer comprising:
a spray nozzle for spraying a drug solution;
a barrel having a distal end, a proximal end, and a cavity extending along an axis connecting the distal end and the proximal end, the spray nozzle being attached to the distal end;
a finger grip provided at the proximal end of the barrel;
a gasket disposed inside the barrel and slidable inside the barrel;
a rod provided at a proximal end of the gasket and having a distal end inserted into the cavity of the barrel; and
a plunger slidably attached to the rod and capable of contacting the rod and pressing the rod toward a distal side,
the plunger includes a contact member which is brought into contact with the finger grip, and a first pressing surface provided at a proximal end thereof,
the rod includes a second pressing surface provided at a proximal end thereof,
the contact member is brought into contact with the finger grip to restrict the movement of the plunger toward the distal side with the gasket positioned between the distal end and the proximal end of the barrel.

2. The sprayer according to claim 1, wherein
at least one of the first pressing surface and the second pressing surface is marked with a order for an operator to press.

3. The sprayer according to claim 1 or claim 2, wherein
the first pressing surface and the second pressing surface are provided close to each other, and
an area of the second pressing surface is larger than an area of the first pressing surface.

4. The sprayer according to any one of claims 1 to 3, wherein
the first pressing surface is positioned closer to a proximal side than the second pressing surface.
